# EUROPEAN PATENT APPLICATION

(11) **EP 4 483 723 A1**
(43) Date of publication of application: **01.01.2025**
(21) Application number: 24185518.8
(22) Date of filing: 28.06.2024
(51) Int. Cl.: A23L 2/58, A23L 2/60, A23L 2/66, A23L 11/60, A23L 25/00, A23L 33/185, A23L 33/21, A61P 3/06, A61P 3/10

(54) **PLANT-BASED PROTEIN DRINK**

(30) Priority: 29.06.2023 DK PA202330109
(71) Applicant: Nature Protein ApS, 4180 Sorø (DK)
(72) Inventor: Elmvang Stenfeldt, Morten, 4180 Sorø (DK)
(74) Representative: Larsen & Birkeholm A/S

(57) **Abstract**

The present invention relates to a method for providing a nutritional composition, the method comprises the steps of (i) providing a first mixture comprising (a) at least one source of protein, and (b) a beta-glucan compound; and (ii) subjecting the first mixture to a heat treatment at a treatment temperature and a treatment time combination allowing one or more chemical reactions of the first mixture, providing the nutritional composition.

## Description

### Technical field of the invention

The present invention relates to process for producing a nutritional composition, and to a nutritional composition as such. In particular the present invention relates to a process for providing a nutritional composition comprising proteins and fibres and having health benefits.

### Background of the invention

Nutritional compositions comprising protein ingredient (often provided from milk, whey or other animal sources) and fibre materials is having increasing interests as they show a range of health benefits for humans digesting the nutritional compositions.

However, conventional nutritional compositions are provided with a significant amount of sugar to provide energy and flavour to the composition.

In order to stabilize and emulsify the nutritional composition compounds like Soy Lecithin, Sunflower Lecithin, mono and diglycerides and guar gum are traditionally used.

The disadvantage of these stabilizers and emulsifiers may be that they may cause various side effects, like intestinal discomfort, constipation, pain in the stomach, flatulence, affected stool, etc.

Hence, there is a need in the industry for an improved nutritional composition, in particular a vegetable nutritional composition, not using animal ingredients, which meets the disadvantages of the traditional nutritional compositions, and which may be easily produced, and which may have a high nutritional value.

### Summary of the invention

Thus, an object of the present invention relates to a nutritional composition, and to a nutritional composition as such, where the nutritional composition comprising proteins and fibres and has health benefits.

In particular, it is an object of the present invention to provide a process and a composition that solves the above-mentioned problems of the prior art with animal ingredients and sweetness, taste and nutritional value.

Thus, one aspect of the present invention relates to a method for providing a nutritional composition, the method comprises the steps of:
i) providing a first mixture comprising:
   a. at least one source of protein;
   b. a beta-glucan compound; and
ii) subjecting the first mixture to a heat treatment at a treatment temperature and a treatment time combination allowing one or more chemical reactions of the first mixture, providing the nutritional composition.

Another aspect of the present invention relates to a nutritional composition comprising:
- at least one source of protein,
- a beta-glucan compound, and
- one or more reaction products from caramelization reactions and/or Maillard reactions.

Yet another aspect of the present invention relates to a nutritional composition according to the present invention, for use in reducing the concentration of blood cholesterol in a mammal and/or for reducing post-prandial glycaemic responses in a mammal.

Still another aspect of the present invention relates to provide a nutritional composition for patients suffering from diabetes, in particular patients suffering from diabetes type-II, and high cholesterol levels.

A further aspect for the present invention relates to a nutritional composition assisting patients suffering from diabetes, and in particular patients suffering from diabetes type-II in controlling the blood sugar level and reducing post-prandial glycaemic responses.

An even further aspect of the present invention relates to the use of one or more reaction products from caramelization reactions and/or Maillard reactions of a source of protein as a sweetener, a flavour and/or an aroma compound in a nutritional composition.

The present invention will now be described in more detail in the following.

### Detailed description of the invention

Accordingly, nutritional compositions, in particular nutritional compositions in the form of beverages, and protein-drinks, comprises large amounts of sugar and proteins mainly from milk or whey product which may be undesirable to many people.

Hence, the inventor of the present invention surprisingly found a way to provide aromas and flavours from reacting in particular the proteins and the carbohydrates.

Thus, a preferred embodiment of the present invention relates to a method for providing a nutritional composition, the method comprises the steps of:
iii) providing a first mixture comprising:
   a. at least one source of protein;
   b. a beta-glucan compound; and
iv) subjecting the first mixture to a heat treatment at a treatment temperature and a treatment time combination allowing one or more chemical reactions of the first mixture, providing the nutritional composition.

The nutritional composition may for oral digestion by a mammal, preferably a human being.

The nutritional composition be provided as a beverage, a porridge, a pudding, or a powder. Preferably, the nutritional composition may be provided a beverage.

The beverage may be a drink which may be a liquid intended for human consumption. The beverage according to the present invention may be provided for satisfying thirst, and nutritional intake.

Preferably, the beverage is a non-alcoholic beverage.

The nutritional composition (or preferably the beverage) may comprise a moisture content in the range of 50-95% (w/w) moisture, such as in the range of 60-92% (w/w), e.g. in the range of 70-90% (w/w), such as in the range of 75-88% (w/w), e.g. in the range of 80-85% (w/w).

In an embodiment of the present invention the nutritional composition according to the present invention may be a ready to drink beverage.

In another embodiment of the present invention the nutritional composition according to the present invention may be a beverage, a protein-drink, or a powder.

In an embodiment of the present invention wherein the nutritional composition is a powdered nutritional composition. The powdered nutritional composition may be mixed with an aqueous solution before ingestion.

The powdered nutritional composition may be provided by spin flash drying, spray drying, freeze drying, or similar gentle drying methods.

The aqueous solution mixed with the powdered nutritional composition may be water, yogurts, juice, smoothies, coffee, tea, or the like. Mixing with water may be preferred.

In an embodiment of the present invention the one or more chemical reactions of the first mixture may include at least one caramelization reaction and/or one or more Maillard reaction or Maillard browning.

Caramelization may be a process of browning of sugar used extensively in cooking for the resulting sweet nutty flavour and brown colour. The brown colours are produced by three groups of polymers: caramelans (C₂₄H₃₆O₁₈), caramelens (C₃₆H₅₀O₂₅), and caramelins (C₁₂₅H₁₈₈O₈₀). As the process occurs, volatile chemicals such as diacetyl are released, producing the characteristic caramel flavour.

Like the Maillard reaction, caramelization may be a type of non-enzymatic browning. Unlike the Maillard reaction, caramelization may be pyrolytic, as opposed to being a reaction with amino acids.

When caramelization involves the disaccharide sucrose, it may be broken down into the monosaccharides fructose and glucose.

The Maillard reaction may be a chemical reaction between amino acids and reducing sugars that gives browned colour and a distinctive flavour.

The reactive carbonyl group of the sugar reacts with the nucleophilic amino group of the amino acid and forms a complex mixture of poorly characterized molecules that may be responsible for a range of aromas and flavours.

During heating, the Maillard reactions may produce hundreds of different flavour compounds depending on the chemical constituents, the temperature, the cooking time, and the presence of air. These compounds, in turn, often break down to form yet more flavour compounds.

The inventor of the present invention surprisingly found that by controlling the development of aromas and flavours (e.g. from the caramelization reaction and the the Maillard reaction) the addition of sugars and sweeteners to the nutritional composition according to the present invention may be avoided or significantly reduced.

In an embodiment of the present invention the heat treatment may include a treatment temperature in the range of 110-180°C.

The heat treatment may include a treatment temperature in the range of 100-200°C, such as in the range of 110-180°C, e.g. in the range of 120-160°C, such as in the range of 130-150°C, e.g. in the range of 135-145°C, such as about 140°C.

The heat treatment may be performed for a period (treatment time) of 1 second to 5 minutes, such as in the period of 3 second to 3 minutes, e.g. in the period of 5 seconds to 2 minutes, such as in the period of 7 second to 1 minutes, e.g. in the period of 10 seconds to 50 seconds, such as in the period of 12 seconds to 40 seconds, e.g. in the period of 15 seconds to 30 seconds, such as in the period of 18 seconds to 25 seconds.

The heat treatment may be provided at a treatment temperature and a treatment time combination allowing one or more chemical reactions of the first mixture. This treatment temperature and treatment time combination may be as the treatment temperature increases the treatment time decreases or a when the treatment time increases the treatment temperature decreases.

In an embodiment of the present invention the heat treatment may be a single heat treatment, including one fixed treatment temperature and one fixed treatment time.

In another embodiment of the present invention the heat treatment may be multiple heat treatment, including two or more treatment temperatures and one or more treatment times.

By changing and controlling the heat treatment (e.g. by changing and controlling the treatment temperature and the treatment time) different chemical reactions may be provided resulting in different flavour and aroma compounds suitable for the present nutritional composition.

In an embodiment of the present invention the heat treatment may be provided at a treatment temperature and a treatment time combination with a treatment temperature in the range of 100-200°C, such as in the range of 110-180°C, e.g. in the range of 120-160°C, such as in the range of 130-150°C, e.g. in the range of 135-145°C, such as about 140°C; for a period of 1-40 seconds, such as for a period of 2-20 seconds, e.g. for a period of 3-15 seconds, such as for a period of 4-10 seconds, e.g. for a period of 2-5 seconds, such as for a period of 3-4 seconds.

In an embodiment of the present invention method for providing the nutritional composition involves a controlled development of one or more chemical reactions of the first mixture providing the nutritional composition.

Preferably, the method according to the present invention (and/or the controlled development of the one or more chemical reaction) does not involve microbial fermentation.

The method according to the present invention (and/or the controlled development of the one or more chemical reaction) does not involve addition of one or more enzymes for providing chemical reactions, flavour compounds or aroma compounds in the nutritional composition.

The first mixture may be subjected to mixing before being heat treated.

The mixing may include vigorously mixing. Preferably, mixing may be performed by high-shear mixing and/or homogenization.

High-shear mixing may disperse, or transport, the solid particles of the first mixture (e.g. the source of protein and the beta-glycan compound) into a main continuous liquid phase. The mixing of the first mixture may result in a suspension comprising a heterogenous mixture of solids and liquid.

High-shear mixing may preferably be performed until the ingredients in the nutritional composition may be dispersed and hydrated. The dispersion and hydration may take 10-90 minutes, such as for a period of 20-75 minutes, e.g. for a period of 30-50 minutes, such as for a period of about 45 minutes.

In an embodiment of the present invention shear mixing may be performed at reduced pressure. Preferably, the shear mixing may be performed at vacuum.

Homogenization of the first mixture may be a process including a chemical or a physical treatment by which the first mixture (comprising the source of protein and the beta-glycan compound) into a uniform composition that does not, or substantially not, separate when stored.

In an embodiment of the present invention the first mixture, the nutritional composition or both the first mixture and the nutritional composition may be subjected to homogenisation.

Preferably, the first mixture may be subjected to homogenization.

The homogenized first mixture may be subjected to heat treatment as described in step (ii).

Homogenization may be performed at elevated temperatures, such as a temperature in the range of 30-95°C, e.g. in the range of 50-90°C, such as in the range of 60-85°C, e.g. in the range of 65-80°C, such as in the range of 70-75°C.

Homogenization may be performed at a pressure in the range of 100-300 bar, such as at 125-250 bar, e.g. at 150-225 bar, such as at 175-200 bar; for a period of 10-100 minutes, such as for a period of 25-75 minutes, e.g. for a period of 40-60 minutes, such as for about 50 minutes.

In an embodiment of the present invention the source of protein may comprise a protein content of at least 2 g protein/100 g source of protein, such as at least 2.2 g protein/g source of protein, e.g. at least 2.4 g protein/100 g source of protein, such as at least 2.6 g protein/g source of protein, e.g. at least 2.8 g protein/100 g source of protein, such as at least 3.0 g protein/g source of protein, e.g. at least 3.2 g protein/100 g source of protein, such as at least 3.4 g protein/g source of protein, e.g. at least 3.6 g protein/100 g source of protein, such as at least 3.8 g protein/g source of protein, e.g. at least 4.0 g protein/100 g source of protein, such as at least 4.2 g protein/g source of protein, e.g. at least 4.4 g protein/100 g source of protein, such as at least 4.6 g protein/g source of protein, e.g. at least 4.8 g protein/100 g source of protein, such as at least 5.0 g protein/g source of protein, e.g. at least 5.2 g protein/100 g source of protein.

In another embodiment of the present invention, the source of protein may comprise at least 10% (w/w) protein, such as at least 12% (w/w) protein, e.g. at least 14% (w/w) protein, such as at least 16% (w/w) protein, e.g. at least 18% (w/w) protein, such as at least 20% (w/w) protein, e.g. at least 22% (w/w) protein, such as at least 24% (w/w) protein, e.g. at least 26% (w/w) protein, such as at least 28% (w/w) protein, e.g. at least 30% (w/w) protein.

The at least one source of protein may preferably be selected from an animal material, a source of protein or a combination hereof.

Preferably the animal material may comprise milk, whey, casein, or other milk fraction.

In an embodiment of the present invention the at least one source of protein may comprise a source of protein.

Preferably, the vegetable is a legume.

The source of protein may not be a cereal material.

The source of protein may be selected from the family *Fabaceae.* Preferably, the family *Fabaceae* may be the genus Pisum.

In an embodiment of the present invention the source of protein may be selected from peas, beans, fava beans, soybeans, chickpeas, peanuts, lentils, lupins bean, hemp, grass, rice, alfalfa, pumpkin seeds, quinoa, chia, sunflower seeds, or a combination hereof, preferably the source of protein may be peas, fava beans, soybeans og chickpeas. Even more preferably, the source of protein may be peas.

The source of protein may comprise a combination of a legume and a cereal material.

The cereal material may be selected from oat, wheat, rye, barley, rice, millet and/or maize.

In an embodiment of the present invention the source of protein may comprise the combination of:
- peas, beans, fava beans, soybeans, chickpeas, peanuts, lentils, and/or lupins bean; in combination with
- oat, wheat, rye, barley, rice, millet and/or maize.

In particular the source of protein may comprise the combination of peas and wheat (in particular wheat bran).

When the source of protein comprises the combination of a legume and a cereal material, the legume may constitute 2-90% (w/w) on a dry-matter basis, of the combined source of protein, such as in the range of 5-80% (w/w), e.g. in the range of 10-75% (w/w), such as in the range of 15-70% (w/w), e.g. in the range of 20-65% (w/w), such as in the range of 30-60% (w/w), e.g. in the range of 40-55% (w/w), such as about 50% (w/w).

The nutritional composition comprises in the range of 2-80% (w/w) source of protein on a dry-matter basis, such as in the range of 5-75% (w/w), e.g. in the range of 10-70% (w/w), such as in the range of 15-60% (w/w), e.g. in the range of 20-55% (w/w) such as in the range of 30-50% (w/w), e.g. about 40% (w/w)

The at least one source of protein may be added to the nutritional composition as a protein source of the nutritional composition. Thus, it may be preferred that a source of protein having a high protein content may be used.

In addition to the at least one source of protein, the first mixture also comprises a glucan compound.

A glucan compound may be a polysaccharide derived from D-glucose, linked by glycosidic bonds. Glucans may be noted in two forms: alpha-glucans and beta-glucans. Many beta-glucans may be considered medically important. They may represent a drug target for antifungal medications of the echinocandin class.

The alpha-glucans may be selected from:
dextran, α-1,6-glucan with α-1,3-branches
floridean starch, α-1,4- and α-1,6-glucan
glycogen, α-1,4- and α-1,6-glucan
pullulan, α-1,4- and α-1,6-glucan
starch, a mixture of amylose and amylopectin, both α-1,4- and α-1,6-glucans

The beta-glucan may be selected from:
cellulose, β-1,4-glucan
chrysolaminarin, β-1,3-glucan
curdlan, β-1,3-glucan
laminarin, β-1,3- and β-1,6-glucan
lentinan, a strictly purified β-1,6:0-1,3-glucan from Lentinus edodes
lichenin, β-1,3- and β-1,4-glucan
oat beta-glucan, β-1,3- and β-1,4-glucan
pleuran, β-1,3- and β-1,6-glucan isolated from Pleurotus ostreatus
zymosan, β-1,3-glucan

In a preferred embodiment of the present invention the first mixture also comprises a beta-glucan compound in addition to the at least one source of protein.

The beta-glucan compound may act as emulsifier and/or stabilizer for the nutritional composition according to the present invention resulting in a smoother, homogenous, and/or more appealing texture or indulgent, creamy mouthfeel of the nutritional composition and/or the drink, beverage, porridge, or pudding, of the present invention.

Preferably, the glucan, in particular the beta-glucan according to the present invention may substitute traditional stabilizers and emulsifiers like Soy Lecithin, Sunflower Lecithin, mono and diglycerides and guar gum which are traditionally used in the industry.

In an embodiment of the present invention the nutritional composition according to the present invention does not comprise a Soy Lecithin, Sunflower Lecithin, mono and diglycerides or guar gum.

Beta-glucan may be arranged in six-sided D-glucose rings connected linearly via a 1-3 glycosidic link in the backbone. Although technically beta-glucans may include chains of D-glucose polysaccharides linked by beta-type glycosidic bonds, by convention not all beta-D-glucose polysaccharides are categorized as beta-glucans compounds according to the present invention. In the present context cellulose may preferably not be considered a beta-glucan compounds according to the present invention, e.g. as it is insoluble in water.

In an embodiment of the present invention the beta-glucan compound may be one or more cereal beta-glucan, one or more beta-glucan extract, one or more beta-glucan concentrate or a combination hereof.

Preferably, the beta-glucan compound may be one or more beta-glucan extract.

The beta-glucan extract may comprise a high-concentration of soluble fibers.

Preferably, the beta-glucan extract comprises at least 15%(w/w) soluble beta-glucan fibers, such as at least 20% (w/w) soluble fibers, e.g. at least 25% (w/w) soluble beta-glucan fibers, such as at least 30% (w/w), e.g. at least 35% (w/w), such as at least 40% (w/w), e.g. at least 45% (w/w), such as at least 50% (w/w), e.g. at least 55% (w/w), such as at least 60% (w/w), e.g. at least 65% (w/w), such as at least 70% (w/w), e.g. at least 75% (w/w), such as at least 80% (w/w), e.g. at least 85% (w/w), such as at least 90% (w/w), e.g. at least 95% (w/w), such as at least 98% (w/w).

In an embodiment of the present invention the beta-glucan compound comprises very little amounts of insoluble fibers. Preferably, the beta-glucan compound comprises less than 20% (w/w) insoluble fibers, such as less than 15% (w/w) insoluble fibers, e.g. less than 10% (w/w) insoluble fibers, such as less than 5% (w/w) insoluble fibers, e.g. less than 4% (w/w) insoluble fibers, such as less than 3% (w/w) insoluble fibers, e.g. less than 2% (w/w) insoluble fibers, such as less than 1% (w/w) insoluble fibers.

In an embodiment of the present invention the nutritional composition comprises no or very little amounts of alpha-glucan fibers. Preferably, the nutritional composition comprises less than 20% (w/w) alpha-glucan fibers, such as less than 15% (w/w) alpha-glucan fibers, e.g. less than 10% (w/w) alpha-glucan fibers, such as less than 5% (w/w) alpha-glucan fibers, e.g. less than 4% (w/w) alpha-glucan fibers, such as less than 3% (w/w) alpha-glucan fibers, e.g. less than 2% (w/w) alpha-glucan fibers, such as less than 1% (w/w) alpha-glucan fibers.

Alpha-glucan fibers may include dextran (α-1,6-glucan with α-1,3-branches), floridean starch (a-1,4- and α-1,6-glucan), glycogen (a-1,4- and α-1,6-glucan), pullulan (a-1,4- and α-1,6-glucan), and/or starch, e.g. a mixture of amylose and amylopectin (both α-1,4- and α-1,6-glucans).

In yet an embodiment of the present invention the nutritional composition comprises no or very little amounts of cellulose. Preferably, the beta-glucan compound comprises less than 20% (w/w) cellulose, such as less than 15% (w/w) cellulose, e.g. less than 10% (w/w) cellulose, such as less than 5% (w/w) cellulose, e.g. less than 4% (w/w) cellulose, such as less than 3% (w/w) cellulose, e.g. less than 2% (w/w) cellulose, such as less than 1% (w/w) cellulose.

In a further embodiment of the present invention the nutritional composition comprises no or very little amounts of cellulose. Preferably, the beta-glucan compound comprises less than 20% (w/w) cellulose, such as less than 15% (w/w) cellulose, e.g. less than 10% (w/w) cellulose, such as less than 5% (w/w) cellulose, e.g. less than 4% (w/w) cellulose, such as less than 3% (w/w) cellulose, e.g. less than 2% (w/w) cellulose, such as less than 1% (w/w) cellulose.

In an embodiment of the present invention the beta-glucan compound may be a gluten-free beta-glucan compound.

In an embodiment of the present invention the beta-glucan compound may be extracted from a plant material or an algae material, providing the beta-glucan extract. Preferably, the plant material may be a cereal material, like oat, wheat, rye, barley, rice, millet and/or maize.

Preferably, the beta-glucan compound may be extracted from the bran of the cereal material, resulting in the beta-glucan extract.

In an embodiment of the present invention the beta-glucan compound comprises a protein content below 15% (w/w), e.g. below 12% (w/w), such as below 10% (w/w), e.g. below 8% (w/w), such as below 6% (w/w), e.g. below 4% (w/w), such as below 3% (w/w), e.g. below 2% (w/w), such as below 1% (w/w).

The nutritional composition according to the present invention comprises a glucan compound, Preferably, the glucan compound according to the present invention consists essentially of a beta-glucan compound.

In the context of the present invention, the terms "consisting essentially of" and "consists essentially of", relate to a limitation of the scope of a claim to the specified features or steps, and those features or steps, not mentioned and which do not materially affect the basic and novel characteristic(s) of the claimed invention.

The amount of glucan compound, in particular beta-glucan compound, may be provided in a concentration in the range of 1-30 g/l of the nutritional composition (or the beverage), such as in the range of 2-20 g/l, e.g. in the range of 3-15, such as in the range of 4-12 g/l, e.g. in the range of 5-10, such as in the range of 6-9 g/l, e.g. in the range of 7-8.

Preferably, the nutritional composition comprises at least 1% (w/w) beta-glucan compound relative to the nutritional composition.

In an embodiment of the present invention the nutritional composition comprises at least 1% (w/w) glucan compound, in particular beta-glucan compound, relative to the nutritional composition, such as at least 2% (w/w), e.g. at least 3% (w/w), such as at least 4% (w/w), e.g. at least 5% (w/w), such as at least 6% (w/w), e.g. at least 7% (w/w), such as at least 8% (w/w), e.g. at least 9% (w/w), such as at least 10% (w/w), e.g. at least 12% (w/w), such as at least 15% (w/w), e.g. at least 18% (w/w), such as at least 20% (w/w), e.g. at least 25% (w/w), such as at least 30% (w/w), such as in the range of 1-30% (w/w), e.g. in the range of 2-20% (w/w), such as in the range of 3-10% (w/w), e.g. in the range of 3.5-5% (w/w), such as in the range of 3.5-4% (w/w).

The one or more source of protein may be subjected to a vegetable pre-treatment before added to the first mixture.

The vegetable pre-treatment may include grinding or milling to provide a finely divided source of protein.

In an embodiment of the present invention the finely divided source of protein may have a particle size D90 below 2000 µm, such as below 1500 µm, e.g. below 1000 µm, such as below 800 µm, e.g. below 600 µm, such as below 500 µm, e.g. below 400 µm, such as below 300 µm, e.g. below 200 µm, such as below 100 µm, e.g. below 75 µm, such as below 50 µm, e.g. below 40 µm, such as below 30 µm, e.g. below 25 µm, such as below 20 µm, e.g. below 15 µm.

The finely divided source of protein may have a particle size D90 in the range of 1-2000 µm, such as in the range of 5-1000 µm, e.g. in the range of 10-500 µm, such as in the range of 15-300 µm, e.g. in the range of 20-100 µm, such as in the range of 30-75 µm, e.g. in the range of 40-50 µm.

The glucan compound, in particular the beta-glucan compound, may be subjected to a glucan pre-treatment before added to the first mixture.

The glucan pre-treatment may include grinding or milling to provide a finely divided glucan material.

The finely divided glucan material may have a particle size D90 below 2000 µm, such as below 1500 µm, e.g. below 1000 µm, such as below 800 µm, e.g. below 600 µm, such as below 500 µm, e.g. below 400 µm, such as below 300 µm, e.g. below 200 µm, such as below 100 µm, e.g. below 75 µm, such as below 50 µm.

The finely divided source of protein may have a particle size D90 in the range of 50-2000 µm, such as in the range of 75-1000 µm, e.g. in the range of 100-500 µm, such as in the range of 150-300 µm, e.g. in the range of 200-275 µm, such as in the range of 210-250 µm, e.g. above 220 µm.

A preferred embodiment of the present invention relates to a nutritional composition comprising:
- at least one source of protein,
- a beta-glucan compound, and
- one or more reaction products from caramelization reactions and/or Maillard reactions.

The one or more reaction products may be obtained from the one or more chemical reactions of the first mixture (preferably the caramelization reactions and/or Maillard reactions) providing the nutritional composition.

The one or more reaction products from caramelization reactions and/or Maillard reactions may be resulting in a sweet and optionally nutty flavour whereby reduced amount of sugar and sweetener needs to be added to the nutritional composition to provide the desired taste.

The one or more reaction products from caramelization reactions and/or Maillard reactions may also result in a brown or brownish colour.

The strength of the brown or brownish colour obtained from the one or more reaction products from caramelization reactions and/or Maillard reactions may be detectable by suitable analytical means known to the skilled person. In an embodiment of the present invention, colour (e.g. the brown or brownish colour) obtained from the one or more reaction products from the caramelization reactions and/or the Maillard reactions may not be visually inspected by the human eye.

In another embodiment of the present invention, the colour (e.g. the brown or brownish colour) obtained from the one or more reaction products from the caramelization reactions and/or the Maillard reactions may be visually inspected by the human eye. The skilled person would know that the visual inspection would also depend on the other ingredients present in the nutritional composition that may influence or cover the colour developed by the caramelization reactions and/or the Maillard reactions.

The one or more reaction products from caramelization reactions may include caramelans (e.g. C₂₄H₃₆O₁₈), caramelens (e.g. C₃₆H₅₀O₂₅), caramelins (C₁₂₅H₁₈₈O₈₀), volatile chemicals (such as diacetyl), or a combination hereof.

The one or more reaction products from the Maillard reactions may include glycation products, which may be a covalent attachment of a carbohydrate (in particular glucose, fructose, and their derivatives) to a protein, a lipid or a nucleic acid molecule. Preferably, the glycation products according to the present invention may relate to a covalent attachment of carbohydrates (in particular glucose, fructose, and their derivatives) to a protein.

The degree of the reaction products from caramelization reactions and/or the reaction products from the Maillard reactions may depend on (and be controlled by) the heat treatment provided in step (ii).

The nutritional composition according to the present invention may be a beverage or used in a beverage having a moisture content in the range of 2-25% (w/w), e.g. in the range of 3-20% (w/w), such as in the range of 4-15% (w/w), e.g. in the range of 5-12% (w/w), such as in the range of 6-10% (w/w), e.g. in the range of 7-9% (w/w), such as about 8% (w/w).

From a nutritional standpoint, too much or too little amount of water in the nutritional composition or the beverage may not have any negative impact. The nutritional impact of the nutritional composition or the beverage may be affected by the amount of protein and/or beta-glucan ingested.

In an embodiment of the present invention the first mixture, or the nutritional composition, may be provided with one or more sugars, one or more sweeteners, or a combination hereof.

In an embodiment of the present invention one or more sweeteners may be stevia, aspartame, erythritol, saccharin, sucralose, cyclamate, Acesulfame K, Neotame, Monk fruit extract, or a combination hereof.

The one or more sugar may be selected from glucose, fructose, sucrose, maltose or a combination hereof.

The nutritional composition may comprise less than 100 g/l sugar (added sugar and/or added sweetener), such as less than 90 g/l, e.g. less than 80 g/l, such as less than 70 g/l, e.g. less than 60 g/l, such as less than 50 g/l, e.g. less than 40 g/l, such as less than 30 g/l, e.g. less than 25 g/l, such as less than 20 g/l, e.g. less than 15 g/l, such as less than 10 g/l, e.g. less than 5 g/l, such as less than 4 g/l, e.g. less than 3 g/l, such as less than 2 g/l, e.g. less than 1 g/l.

The nutritional composition according to the present invention may comprise a reduced Glycemic Index, and preferably a low Glycemic Index, compared to other similar products.

The Glycemic Index measures the impact of carbohydrates on human or animal blood sugar level. Foods and beverages are given a number from 0 to 100 based on how quickly they cause human blood sugar (or the animal blood sugar) to rise after eaten the food or drinking the beverage.

Foods and beverages with a high Glycemic Index can cause rapid spikes in human (or animal) blood glucose level, while those with lower GIs have less of an effect on the human (or animal) blood glucose level.

A low glycemic index diet may be an eating plan that focuses on foods with a low GI rating. The goal of such diets may be to help people maintain healthy blood sugar levels and reduce their risk for diabetes, heart disease, and other health problems.

Furthermore, when it comes to managing weight loss or preventing chronic diseases related to poor nutrition choices, the change of (at least some) high-glycemic carbs for healthier options, with low glycemic carbs, can be beneficial over time because these types of food digest slower, and therefore, does not spike human (or animal) insulin levels as much, which may lead to better control over hunger cravings throughout the day helping keep energy stable instead of having sudden highs followed by lows due fatigue caused by hypoglycemia when sugars drop too fast post meal consumption - this kind of response may ultimately affects mood swings negatively too.

Preferably, the Glycemic Index of the nutritional composition according to the present invention may be below 75, such as below 70, e.g. below 65, such as below 60, e.g. below 55, such as below 50, e.g. below 45, such as below 40, e.g. below 35, such as below 30 e.g. below 25, such as below 20.

In an embodiment of the present invention the nutritional composition may comprise a total fat content in the range of 0.1-20 g/l, such as in the range of 0.5-18 g/l, e.g. in the range of 1-15 g/l, such as in the range of 5-12 g/l, e.g. about 10 g/l. Preferably, at least 25% of the fat in the nutritional composition may be monounsaturated fat or polyunsaturated fat, such as at least 30%, e.g. at least 40%, such as at least 50%, e.g. at least 60%, such as at least 70%, e.g. at least 80%, such as at least 90%, e.g. in the range of 25-90%, such as in the range of 40-80%, e.g. in the range of 50-70%.

Preferably, the nutritional composition according to the present invention comprises a calories content per 100 ml in the range of 25-85 kcal, e.g. in the range of 35-70 kcal, such as in the range of 40-65 kcal, e.g. in the range of 45-60 kcal.

The first mixture and/or the nutritional composition may be provided with a flavoring agent. The flavoring agent may be a natural flavoring agent or an artificial flavoring agent. Preferably, the flavoring agent may be a natural flavoring agent.

The flavoring agent may be selected from cocoa, chocolate, vanilla, caramel, hazelnut, mocha, milky chocolate, strawberry, lemon, lime, blueberry, raspberry, or coco.

The first mixture and/or the nutritional composition may be provided with a colorizing agent. The colorizing agent may be a natural colorizing agent or an artificial colorizing agent. Preferably, the colorizing agent may be a natural colorizing agent.

The flavoring agent may also be used for colorizing the nutritional composition. If a different color of the nutritional composition is intended, compared to the color provide by the nutritional composition as such (without the presence of a flavoring agent) and different from the color provided by the flavoring agent, a separate colorizing agent may be added. Preferably, the separate colorizing agent may be a natural colorizing agent. The skilled person knows the colorizing agents suitable for the nutritional composition according to the present invention.

Functional proteins or peptides, vitamins, minerals, oils (preferably, vegetable oils and/or unsaturated oils), and/or probiotics may be added to the first mixture.

In an embodiment of the present invention the flavoring agent, the colorizing agent, the functional proteins or peptides, the vitamins, the minerals, the oils (preferably, the vegetable oils and/or the unsaturated oils), and/or the probiotics may be added to the nutritional composition after homogenization (if homogenized) and heat treatment, in order to preserve structure, function and/or activity.

The nutritional composition may comprise one or more of the following amino acids:
- Histidine; preferably, comprising 10-30 mg histidine/g protein, preferably 15-25 mg histidine/g protein, more preferably 18-22 mg histidine/g protein, even more preferably about 20 mg histidine/g protein; and/or
- Isoleucine; preferably, comprising 30-50 mg isoleucine/g protein, preferably 35-45 mg isoleucine/g protein, more preferably 38-42 mg isoleucine/g protein, even more preferably about 40 mg isoleucine/g protein; and/or
- Leucine; preferably, comprising 60-80 mg leucine/g protein, preferably 65-75 mg leucine/g protein, more preferably 67-72 mg leucine/g protein, even more preferably about 69 mg leucine/g protein; and/or.
- Lysine; preferably, comprising 40-60 mg lysine/g protein, preferably 45-55 mg lysine/g protein, more preferably 47-52 mg lysine/g protein, even more preferably about 50 mg lysine/g protein; and/or
- Methionine; preferably, comprising 2-20 mg methionine/g protein, preferably 5-15 mg methionine/g protein, more preferably 8-13 mg methionine/g protein, even more preferably about 11 mg methionine/g protein; and/or
- Cysteine; preferably, comprising 3-20 mg cysteine/g protein, preferably 6-16 mg cysteine/g protein, more preferably 9-14 mg cysteine/g protein, even more preferably about 12 mg cysteine/g protein; and/or
- Phenylalanine; preferably, comprising 45-65 mg phenylalanine/g protein, preferably 50-60 mg phenylalanine/g protein, more preferably 52-58 mg phenylalanine/g protein, even more preferably about 55 mg phenylalanine/g protein; and/or
- A combination of phenylalanine and tyrosine; preferably, comprising 45-65 mg phenylalanine+ tyrosine/g protein, preferably 50-60 mg phenylalanine+ tyrosine/g protein, more preferably 52-58 mg phenylalanine+ tyrosine/g protein, even more preferably about 55 mg phenylalanine+ tyrosine/g protein; and/or
- Threonine; preferably, comprising 20-40 mg threonine/g protein, preferably 25-35 mg threonine/g protein, more preferably 27-32 mg threonine/g protein, even more preferably about 30 mg threonine/g protein; and/or
- Tryptophane, preferably, comprising 1-20 mg tryptophane/g protein, preferably 5-15 mg tryptophane/g protein, more preferably 7-12 mg tryptophane/g protein, even more preferably about 10 mg tryptophane/g protein; and/or
- Valine; preferably, comprising 35-55 mg valine/g protein, preferably 40-50 mg valine/g protein, more preferably 42-47 mg valine/g protein, even more preferably about 45 mg valine/g protein; or
- Any combination hereof.

Preferably, the nutritional composition comprises an amino acid distribution comprising:
- Lysine; preferably, comprising 40-60 mg lysine/g protein, preferably 45-55 mg lysine/g protein, more preferably 47-52 mg lysine/g protein, even more preferably about 50 mg lysine/g protein; and/or
- Methionine; preferably, comprising 2-20 mg methionine/g protein, preferably 5-15 mg methionine/g protein, more preferably 8-13 mg methionine/g protein, even more preferably about 11 mg methionine/g protein; and/or
- Phenylalanine; preferably, comprising 45-65 mg phenylalanine/g protein, preferably 50-60 mg phenylalanine/g protein, more preferably 52-58 mg phenylalanine/g protein, even more preferably about 55 mg phenylalanine/g protein; and/or
- A combination of phenylalanine and tyrosine; preferably, comprising 45-65 mg phenylalanine+ tyrosine/g protein, preferably 50-60 mg phenylalanine+ tyrosine/g protein, more preferably 52-58 mg phenylalanine+ tyrosine/g protein, even more preferably about 55 mg phenylalanine+ tyrosine/g protein; and/or
- Tryptophane, preferably, comprising 1-20 mg tryptophane/g protein, preferably 5-15 mg tryptophane/g protein, more preferably 7-12 mg tryptophane/g protein, even more preferably about 10 mg tryptophane/g protein; and/or
- Any combination hereof.

Even more preferably, the nutritional composition comprises an amino acid distribution comprising:
- Lysine; preferably, comprising 40-60 mg lysine/g protein, preferably 45-55 mg lysine/g protein, more preferably 47-52 mg lysine/g protein, even more preferably about 50 mg lysine/g protein; and/or
- Methionine; preferably, comprising 2-20 mg methionine/g protein, preferably 5-15 mg methionine/g protein, more preferably 8-13 mg methionine/g protein, even more preferably about 11 mg methionine/g protein; or
- Any combination hereof

In an embodiment of the present invention the nutritional composition may be a prebiotic composition.

The prebiotic composition according to the present invention may selectively stimulate the growth and/or activity of one or a limited number of bacteria in the colon, and thus improves host health.

A preferred embodiment of the present invention relates to a nutritional composition according to the present invention, for use in reducing the concentration of blood cholesterol in a mammal and/or for reducing post-prandial glycaemic responses in a mammal.

Preferably the nutritional composition according to the present invention may be suitable for reducing the concentration of blood cholesterol in a mammal or maintaining the concentration of blood cholesterol in a mammal at a normal level.

Preferably, the concentration of blood cholesterol, in particular LDL-cholesterol, in a mammal may be reduced to (or a normal level of blood cholesterol, in particular LDL-cholesterol, may be) below 6 mmol/L, e.g. 5.5 mmol/L, such as 5.0 mmol/L, e.g. 4.5 mmol/L, such as 4.0 mmol/L, e.g. 3.5 mmol/L, such as 3.0 mmol/L, e.g. 2.5 mmol/L, such as 2.0 mmol/L, e.g. 1.5 mmol/L, such as 1.0 mmol/L, e.g. 0.5 mmol/L.

The level to which the concentration of blood cholesterol, in particular LDL-cholesterol, in a mammal may be reduced to (or what is considered the normal level of blood cholesterol, in particular LDL-cholesterol) may be affected by various factors, like if the person have cardiovascular risk factors such as high blood pressure, pre-existing cardiovascular (heart) disease or diabetes, or it the person smokes. These factors may require lower levels of cholesterol in the blood, in particular LDL cholesterol levels.

The nutritional composition according to the present invention may be a suitable nutritional supplement for patients suffering from diabetes, in particular type-II diabetes.

A preferred embodiment of the present invention relates to a nutritional composition for patients suffering from diabetes, and in particular patients suffering from diabetes type-II.

Preferably, the present invention relates to a nutritional composition assisting patients suffering from diabetes, and in particular patients suffering from diabetes type-II in controlling the blood sugar level and reducing post-prandial glycaemic responses.

A preferred embodiment of the present invention relates to the use of one or more reaction products from caramelization reactions and/or Maillard reactions of a source of protein as a sweetener, a flavour and/or an aroma compound in a nutritional composition.

In an embodiment of the present invention the one or more reaction products from caramelization reactions and/or Maillard reactions of a source of protein may be used as a sweetener, a flavour and/or an aroma compound in a beverage, preferably in a nutritional beverage provided for satisfying thirst, and nutritional intake.

In an embodiment of the present invention the one or more reaction products from caramelization reactions and/or Maillard reactions of a source of protein and of a beta-glucan compound may be used as a sweetener, a flavour and/or an aroma compound in a nutritional compound, such as a beverage, preferably in a nutritional beverage provided for satisfying thirst, and nutritional intake.

It should be noted that embodiments and features described in the context of one of the aspects of the present invention also apply to the other aspects of the invention.

### Examples

### Example 1:

Preparation of a nutritional composition according to the present invention, flavored with cacao.

The following ingredients were mixed to a total volume of 1000 L:
- 3.46 kg per 1000 L beta-glucan (having a particular size (D50) below 200 µm),
- 36.8 kg per 1000 L oat protein powder (having a particular size (D50) below 30 µm),
- 45.33 kg per 1000 L pea protein powder (having a particular size (D50) below 30 µm),
- 15 kg per 1000 L cacao powder,
- 40 kg per 1000 L sugar,
- 4.34 kg per 1000 L Vanilla extract,
- 1.08 kg per 1000 L vanilla flavor,
- 1 L per 1000 L Cacao flavor,
- 1.32 L per 1000 L sweetness flavor,
- 851 L water pr. 1000 L (using local tap water).

All base elements, such as powder and liquids are mixed together in a high shear mixer until the ingredients are dispersed and hydrated. The dispersion and hydration may take 10-90 minutes, depending on the type of high shear mixer, and whether or not the shear mixer in run with reduced pressure, e.g. vacuum. Most often high shear mixing is performed for 30-60 minutes. In the present case the shear mixing was performed for 45 minutes under vacuum.

The mix is aired out and subjected to heat treatment at 140C for 10 seconds to allow caramelization and milliard reaction sugars and proteins to enhance taste and sweetness of the heat treated composition. Subjecting the heat treated product to homogenization at a temperature of 70-75°C and a pressure between 100/20 and 250/80, in particular between 160/50 and 200/50 bar resulting in a nutritional composition comprising an extended shelf-life, and comprising caramelized sugars and increased amount of Maillard reaction product of proteins, enhancing taste, flavor and odor.

The nutritional composition may be aseptically bottling before distributed.

### Example 2

Analytical investigation of the nutritional composition from Example 1.

Beta-glucan added in Example 1 showed to provide a healthy benefit to consumers, starting 2 hours after consumption by slightly lowering the cholesterol level specifically the LDL-level, by 15% after 8 weeks of 1 L daily intake (data not shown). In additionally, the beta-glucan acts as a functional stabilizer and in combination with proteins, the beta-glucan is used as emulsifier.

Approximately 1 gram of beta-glucan is used pr. Portion (0.33 L). The water activity is 0,98 and Ph is 6,9.

The nutritional composition provided in Example 1 comprises the following nutritional content:

| **Nutritional Information** | **pr. 100 ml** |
|---|---|
| Kcal | 57.77 |
| Total Fat | 0.99 |
| Saturated Fat | 0.25 |
| Monounsaturated Fat | 0.25 |
| Polyunsaturated Fat | 0.35 |
| Carbohydrate | 1.62 |
| Sugars | 4.09 |
| Fiber | 1.27 |
| Protein | 6.04 |
| Salt | 0.11 |

The amino acid distribution in the nutritional composition obtained from Example 1 has the following composition:

| **Amino acid profile** | |
|---|---|
| | Mg pr. gram of protein |
| Histidine | 19,935 |
| Isoleucine | 39,275 |
| Leucine | 68,7 |
| Lysine* | 50,215 |
| Methionine* | 11,475 |
| Cysteine | 12,175 |
| Phenylalanine* + Tyrosine | 55,89 |
| Threonine | 29,275 |
| Trytophan* | 9,685 |
| Valine | 44,62 |

### Example 3

Preparation of a nutritional composition according to the present invention, flavored with vanilla.

The following ingredients were mixed to a total volume of 1000 L:
- 10,38 kg per 1000 L beta-glucan (having a particular size (D50) below 200 µm),
- 19,9 kg per 1000 L oat protein powder (having a particular size (D50) below 30 µm),
- 24,75 kg per 1000 L pea protein powder (having a particular size (D50) below 30 µm),
- 25 kg per 1000 L sugar,
- 1 kg sodium chloride
- 4.34 kg per 1000 L Vanilla extract,
- 1.08 kg per 1000 L vanilla flavor,
- 1.32 L per 1000 L sweet vanilla flavor,
- 855 L water pr. 1000 L (using local tap water).

All base elements, such as powder and liquids are mixed together in a high shear mixer until the ingredients are dispersed and hydrated. The dispersion and hydration may take 10-90 minutes, depending on the type of high shear mixer, and whether or not the shear mixer in run with reduced pressure, e.g. vacuum. Most often high shear mixing is performed for 30-60 minutes. In the present case the shear mixing was performed for 45 minutes under vacuum.

The mix is aired out and subjected to heat treatment at 140C for 10 seconds to allow caramelization and milliard reaction sugars and proteins to enhance taste and sweetness of the heat treated composition. Subjecting the heat treated product to homogenization at a temperature of 70-75°C and a pressure between 100/20 and 250/80, in particular between 160/50 and 200/50 bar rresulting in a nutritional composition comprising an extended shelf-life, and comprising caramelized sugars and increased amount of Maillard reaction product of proteins, enhancing taste, flavor and odor.

### Example 4

Analytical investigation of the nutritional composition from Example 3.

Beta-glucan added in Example 3 showed to provide a healthy benefit to consumers, starting 2 hours after consumption by lowering the cholesterol level, specifically the LDL-levels, by 15% after 8 weeks of 0,33 L daily intake (data not shown). In additionally, the beta-glucan acts as a functional stabilizer and in combination with proteins, the beta-glucan is used as emulsifier.

Approximately 3 gram of beta-glucan is used pr. Portion (0.33 L). The Ph is 6,9.

The nutritional composition provided in Example 1 comprises the following nutritional content:

| **Nutritional information** | **pr. 100 ml** |
|---|---|
| Calories | 40,63 |
| Total Fat | 0,72 |
| Saturated Fat | 0,20 |
| Monounsaturated Fat | 0,11 |
| Polyunsaturated Fat | 0,16 |
| Carbohydrate | 2,00 |
| Sugars | 2,60 |
| Fiber | 1,92 |
| Protein | 3,08 |
| Salt | 0,11 |

The 9 essential amino acid distribution in the nutritional composition obtained from Example 3 has the following composition:

| **Amino acid profile** | |
|---|---|
| | Mg pr. gram of protein |
| Histidine | 19,935 |
| Isoleucine | 39,275 |
| Leucine | 68,7 |
| Lysine* | 50,215 |
| Methionine* | 11,475 |
| Cysteine | 12,175 |
| Phenylalanine* + Tyrosine | 55,89 |
| Threonine | 29,275 |
| Trytophan* | 9,685 |
| Valine | 44,62 |

## Claims

1. A method for providing a nutritional composition, the method comprises the steps of:
v) providing a first mixture comprising:
a. at least one source of protein;
b. a beta-glucan compound; and
vi) subjecting the first mixture to a heat treatment at a treatment temperature and a treatment time combination allowing one or more chemical reactions of the first mixture, providing the nutritional composition.

2. The nutritional composition according to claim 1, wherein the at least one source of protein is selected from an animal material, a source of protein or a combination hereof.

3. The nutritional composition according to anyone of the preceding claims, wherein the one or more chemical reactions of the first mixture includes at least one caramelization reaction and/or one or more Maillard reaction or Maillard browning.

4. The nutritional composition according to anyone of the preceding claims, wherein the heat treatment includes a treatment temperature in the range of 110-180°C.

5. The method according to anyone of the preceding claims, wherein the first mixture is subjected to homogenization.

6. The method according to anyone of the preceding claims, wherein the source of protein is selected from peas, beans, fava beans, soybeans, chickpeas, peanuts, lentils, lupins bean, hemp, grass, rice, alfalfa, pumpkin seeds, quinoa, chia, sunflower seeds, or a combination hereof, preferably the source of protein is peas, fava beans, soybeans and chickpeas, even more preferably, the source of protein is peas.

7. The method according to anyone of the preceding claims, the source of protein comprises the combination of:
- peas, beans, fava beans, soybeans, chickpeas, peanuts, lentils, and/or lupins bean; in combination with
- oat, wheat, rye, barley, rice, millet and/or maize.

8. The method according to anyone of claims 2-7, wherein the animal material comprises milk, whey, casein, or other milk fraction.

9. The method according to any of the preceding claims, wherein the nutritional composition comprises at least 1% (w/w) beta-glucan compound relative to the nutritional composition.

10. A nutritional composition comprising:
- at least one source of protein,
- a beta-glucan compound, and
- one or more reaction products from caramelization reactions and/or Maillard reactions.

11. The nutritional composition according to claim 10, wherein the at least one source of protein is selected from an animal material, a source of protein or a combination hereof.

12. The nutritional composition according to claim 10, wherein the at least one source of protein is a source of protein.

13. The nutritional composition according to anyone of claims 10-12, wherein the nutritional composition is a beverage, a protein-drink, or a powder.

14. A nutritional composition according to anyone of claims 10-13, for use in reducing the concentration of blood cholesterol in a mammal and/or for reducing post-prandial glycaemic responses in a mammal.

15. Use of one or more reaction products from caramelization reactions and/or Maillard reactions of a source of protein as a sweetener, a flavour and/or an aroma compound in a nutritional composition.
